# EUROPEAN PATENT APPLICATION

(11) **EP 2 269 545 A1**
(43) Date of publication of application: **05.01.2011**
(21) Application number: 09172913.7
(22) Date of filing: 13.10.2009
(51) Int. Cl.: A61F 7/00

(54) **Therapeutic pad having a data storage tag**

(30) Priority: 03.07.2009 EP 09164542
(71) Applicant: Dewaegenaere, Levi Emmerik A., 2970 s'-Gravenwezel (BE)
(72) Inventor: Dewaegenaere, Levi Emmerik A., 2970 s'-Gravenwezel (BE)
(74) Representative: Vossius & Partner

(57) **Abstract**

The invention provides for a therapeutic pad for attachment to the human or animal body. The pad comprises means for storing data. Further, a therapeutic system is provided. The system comprises a therapeutic pads as discussed above and a hydraulic device. The hydraulic device has a tube adapted to connect the hydraulic device to the pad. (Fig. 1)

## Description

The invention relates to a therapeutic pad which can be controlled by an external system. In particular, the pad may be used to cool the human or animal body or parts thereof, such as extremities, a knee, an elbow, and/or the face.

### FIELD OF THE INVENTION

The present invention is in the technical field of therapeutic treatment and diagnosis of a human or animal body.

More particularly, the present invention is in the technical field of therapeutic treatment of a human or animal body by means of a thermal treatment, which ensures the circulation through the pad of a fluid at controlled and actively regulated temperature, pressure and flow. More particularly, the treatment of the body part is carried out through controlled and actively regulated thermal energy exchange between the pad and the body part to which it is applied, such therapeutic pad containing means for associating thermal treatment-related data with the therapeutic pad.

### BACKGROUND OF THE INVENTION

Particular ailments or diseases require treatment by the application of cold or heat to a body part, which has a particular beneficial medical outcome, according to the treatment protocol applied (e.g. intensity and duration of treatment). In the past, this was accomplished, for example, by applying ice or cold liquid to the affected area for cryotherapy, and applying a heat source, such as a heated towel, for heat therapy.

It has been discovered that using a thermal pad considerably increases the efficiency and convenience of such treatment.

However, in some cases, merely providing a hot or cold fluid through the pad to heat or cool the body via the fluid flow through the pad is not sufficient for example, if the body part is swollen (for example, due to an inflammation), the pressure provided by the pad onto the body part may be too strong or too weak if the pad is in used.

Hence, it would be advantageous to allow the specific treatment of the pad to be modified depending on the type of treatment and characteristics of the patient or his illness.

### OBJECTS OF THE INVENTION

It is an object of the present invention to provide a therapeutic pad which treatment can be controlled.

It is an object of the present invention to provide a controllable therapeutic pad having means for storing data related to the controlling.

It is a further object of this invention to provide a therapeutic pad with means for allowing patient specific data to be associated with the patient and treatment applied.

It is a further object of this invention to allow treatment related data to be stored and analysed efficiently.

Further objects and advantages of the present invention will be disclosed and become apparent from the following description.

### SUMMARY OF THE INVENTION

The present invention relates to a therapeutic pad for attachment to the human or animal body and a therapeutic system comprising the pad and a hydraulic device connectable to the pad.

More specific features for preferred embodiments are set out in the dependent claims.

According to the present invention, a therapeutic pad for attachment to the human or animal body is provided. The pad comprises means for storing data.

In a particular embodiment, the pad is adapted to provide a thermal energy exchange to the human or animal body.

In a particular embodiment, the pad is adapted to be connected to a hydraulic device, particularly via a tube.

In a particular embodiment, the pad comprises internal fluid communication channels adapted to be externally connected to a tube.

In a particular embodiment, the means for storing data is an electronic memory chip.

In a particular embodiment, the memory chip is adapted to contactlessly communicate with an external processing unit.

In a particular embodiment, the memory chip is attached to an outer or inner surface of the pad.

In a particular embodiment, the memory chip is attached to the pad via a tag, particularly via an RF tag.

For example, a contactless communication of the pad may be provided using the RFID (radio frequency identification) technology. RFID is a technology similar in theory to bar code identification. With RFID, the electromagnetic or electrostatic coupling in the RF portion of the electromagnetic spectrum is used to transmit signals. An RFID system consists of an antenna and a transceiver, which read the radio frequency and transfer the information to a processing device, and a transponder or tag, which is an integrated circuit containing the RF circuitry and information to be transmitted. High frequency RFID systems (850 MHz to 950 MHz and 2.4 GHz to 2.5 GHz) offer transmission ranges of more than 25 m. In an RFID system, the transponder that contains the data to be transmitted is the "RF tag". An RF reader/writer module (RFID reader/writer module) is a device that is used to interrogate a RFID tag and/or to generate data thereon. The module has an antenna that emits/receives radio waves; the tag responds by sending back its data.

In a particular embodiment, the memory chip is readable and/or writable by more than one pre-determined processing units.

In a particular embodiment, the data is a medical protocol information and/or a result of a medical treatment and/or an information relating to the performance and/or lifetime of the pad.

In a particular embodiment, the data of the data storage of the therapeutic pad may be particularly medical data related to the treatment of the pad. As an example, a representation of the information set in the data is listed below. The data as grouped below is not only 'read' data but also 'write' data if labeled accordingly. The information set can be any subset of the following information (but not limited to this information only, as other information of interest can be defined and stored):
- Therapeutic Pad Serial Number (S/N)
- Pad type (e.g. knee pad, face pad, shoulder pad)
- Production batch
- Production date
- Patient Name (write, only once)
- Patient unique identifier (in hospitals or treatment facilities where identifiers are used instead of the patient name) (write, only once)
- Protocol Name (name of the protocol used for the treatment of the patient for which the therapeutic pad is used) (write)
- Protocol step 1 parameter set (write)
- Protocol step 2 parameter set (write)
- Protocol identifier (write)
- Timestamp of first connection (write only once)
- Timestamp last event (write)
- Thermal treatment server SN (last thermal treatment server to write on the RF tag) (write)
- Type of last event (e.g. disconnection of therapeutic pad, system message, start of treatment) (write)
- Treatment output data set (e.g. response of human body to treatment, therapeutic pad performance) (write)

In a particular embodiment, the information to be stored can be part of the following classes, but not limited to: patient information, medical treatment and medical protocol information, intermediate and final treatment results information, diagnosis information, information related to the use, quality, performance and lifetime of the therapeutic pad, traceability information for patients and therapeutic pads.

The primary responsibility of the thermal treatment device is to ensure the circulation through the therapeutic pad of a fluid at controlled and actively regulated temperature, pressure and flow, with the stated ultimate goal to ensure positive outcome for the patient being treated. In order to effectively, efficiently, reliably and safely perform its primary responsibility, the thermal treatment device may require an initial input of information and instruction by an operator (for example, the physician), as well as subsequent inflow of real time information collected through some sensors externally placed, for example, in a treatment device, or arranged at suitable location on the patient in order to determined relevant control parameters, like blood pressure or pulse rate.

In a particular embodiment, the pad is a disposable pad.

According to the invention, a therapeutic system is provided. The system comprises one of the therapeutic pads as discussed above and a hydraulic device. The hydraulic device has a tube adapted to connect the hydraulic device to the pad.

In a particular embodiment, the hydraulic device is adapted to be connected to the pad via two tubes.

In a particular embodiment, hydraulic device and the pad are non-removably connected to each other.

In a particular embodiment, the hydraulic device is adapted to be controlled by a medical treatment device.

Because the patient will be moved among different thermal treatment locations (e.g. from the operating room to the recovery room, then to his bedroom, possibly during the day to a physiotherapy facility), and because of the practical impossibility to have the same thermal treatment device used for the same patient in all the above mentioned locations, there is a clear problem in having the relevant information used by the thermal treatment device to follow the patient in a practical way. The invention here described allows the thermal treatment device operator (e.g. the physician, a nurse) to set the initial information needed for executing the treatment (e.g. right treatment protocol). The operator will insert the initial information using the thermal treatment device user interface or some other handheld device or user interface. Due to the compatibility of the therapeutic pad with more than one treatment device, it is possible that the pad is controlled dependent on the data transmitted to and/or from the pad to the active medical treatment device.

In one embodiment, a sensor may be provided and may be adapted to determine the body part related parameter(s), such as the skin conductivity, the skin color, the skin temperature, the skin texture, the heart rate, the blood pressure, the blood saturation.

Additionally the system may comprise means for a controlled supply of medication, e.g., a certain pharmaceutical supplied by a drug pump, to the patient, wherein the system determines - via suitable sensors - the patient's response on the medication, namely the change of the body part related parameter(s). The system may provide a certain diagnosis based on the patient,' response and/or modify the thermal treatment in a controlled manner in order to optimize the patient's treatment by means of a closed loop (feedback) control.

For example, the combination of treatment by the thermal treatment device in conjunction with other medical treatments, such as, but not limited to antibiotics, anti-inflammatory drugs, and chemotherapy drugs, can cause a positive synergistic treatment effect. In one embodiment, the thermal treatment device may by used to apply cryo-treatment to an area of the body (e.g, the arm) which is infected with a bacteria which is, for example, normally resistant to antibiotics (e.g. methicillin-resistant Staphylococcus aureus). The application of cryo-treatment in this way may cause a reduction in bacterial division and/or spread in the tissue, providing more time for the antibiotics to work effectively in the affected area. This may result in a favorable treatment outcome where amputation may have been the only other viable option.

Further, the invention disclosed herein could operate to monitor the patient's progress with regards to cryo-treatment in combination with other medical treatments and update the treatment protocol accordingly, for example, the choice of medicine and optimal dose rate to maximize pharmaceutical efficacy.

The temperature distribution data may be obtained by an IR (infrared) camera and may be displayed to the physician and/or used for feedback control of the thermal treatment. Furthermore, the time lapsed during the thermal treatment may also be taken into account when controlling the thermal treatment based on the determination of the various body part related parameter(s).

While the present invention discusses the use of biofeedback to adapt a treatment protocol, it is advantageous to disclose the optimal means of obtaining a biofeedback reading, which are preferably non-invasive. For example, the amount of thermal exchange between the thermal pad or Peltier thermal exchange element and skin can be determined by using a temperature sensitive surface between such thermal pad or Peltier thermal exchange element and skin. Alternatively, a measurement of power output to the fluid in the thermal pad or the Peltier thermal exchange element itself can provide an indirect determination of thermal exchange with a body. Alternatively, the amount of thermal exchange between a Peltier thermal exchange element and the skin (such Peltier thermal exchange element preferably being constructed in the manned disclosed in US Patent No. 6017337 by Pira) can be determined indirectly by measuring temperature difference between the cold side and hot side of the Peltier element, and the rate of flow of liquid cooling the heat sink on the hot side. Alternatively, a direct measurement of biofeedback (e.g. temperature) from a patient can be obtained by using sensor means to obtain a reading from a drainage catheter, which, for example, has been inserted into the body following knee reconstructive surgery. Although various preferred means of obtaining biofeedback readings have been disclosed herein, other means of obtaining such readings are not excluded.

### BRIEF DESCRIPTION OF THE DRAWINGS

Fig. 1 schematically shows a top view of a therapeutic pad;
Fig. 2 schematically shows a hydraulic device;
Fig. 3 schematically shows a part of a pad having a RF tag;
Fig. 4 schematically shows the components of an external system controlling a therapeutic pad;
Fig. 5 schematically shows an external device for controlling the therapeutic pad;
Fig. 6 schematically shows an external handheld device for reading/writing data of a therapeutic pad; and
Fig. 7 schematically shows the system controlling the operation of a pad.

### DETAILED DESCRIPTION OF THE INVENTION

Various embodiments of the present invention are described hereinafter with reference to the figures. It should be noted that the figures are not drawn to scale and elements of similar structures or functions are represented by like reference numerals throughout the figures. It should also be noted that the figures are only intended to facilitate the description of specific embodiments of the invention. In addition, an aspect described in conjunction with a particular embodiment of the present invention is not necessarily limited to that embodiment and can be practiced in any other embodiments of the present invention.

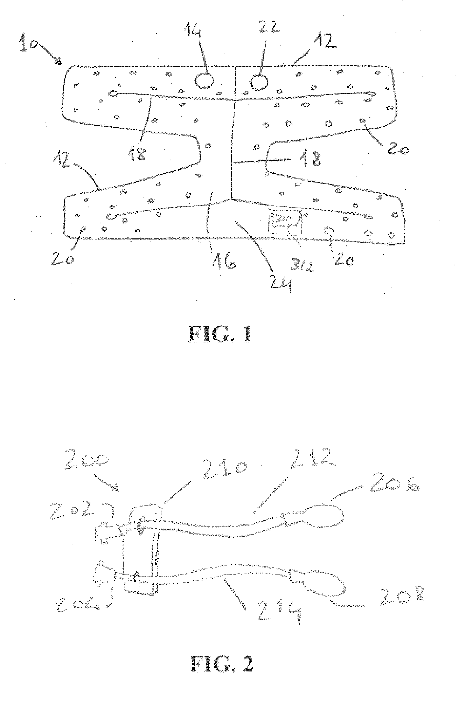

Referring now to the invention in more detail, Fig. 1 shows a top view of a therapeutic pad (10). The shape of the therapeutic pad (10) may be modified to fit to particular part of the body to be treated and depends on the application. The depicted therapeutic pad (10) in Fig. 1 is, for example, for knee application. Other shapes are also possible.

The therapeutic pad (10) is outlined by externally enclosing welding lines (12). The therapeutic pad receives the fluid, for example, from a thermal treatment device through a fluid inlet (14). The fluid circulates in the therapeutic pad through internal communicating channels (16), delimited by internal welding lines (18) and welding points (20). Finally, the fluid returns to the thermal treatment device through a fluid outlet (22).

In Fig. 2 there is shown a top view of a hydraulic device (200). The hydraulic device (200) is formed by two tubes (212, 214), two hydraulic connectors connected to the tubes (212, 214), and two hydraulic sealed connections (206, 208). The hydraulic sealed connections are preferably sealed in a non-removable way to the fluid inlet (14) and outlet (22) of the therapeutic pad (10). Preferably, the hydraulic device (200) is assembled and forms one single part with the therapeutic pad (10). For example, the hydraulic device and the pad may be integrally formed, e.g. integrally molded. Optionally the hydraulic device (200) can be equipped with an element (210) with the mechanical goal of keeping the tubes close to each other. In an alternative but similar embodiment, two connectors may be embedded in such an element (210). The hydraulic device (200) allows the circulation of the fluid from a thermal treatment device through the connectors (202, 204) to and through the therapeutic pad (10).

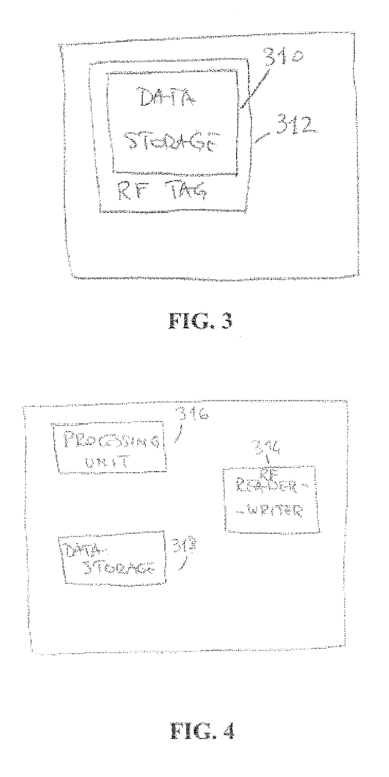

Fig. 3 shows a part of the therapeutic pad having an RF tag (312) attached. In this embodiment, the tag 312 comprises a data storage (310) to allow the storage of data received, e.g., from an external device. In a particular embodiment, the storage is a treatment and patient information data storage and is placed on the therapeutic pad (10) via a tag.

Fig. 4 shows a functional diagram of the components of an external treatment device for controlling the treatment of the pad to the body. In a particular embodiment, the processing unit (316), a data storage (318), and a RF reader and/or writer module (314) form a contactless communication system. For example, if a readable and writeable electronic memory chip, e.g. the data storage (310), is placed in a RF tag (312), see also Fig. 3, and attached on or within a part of the pad, the contactless communication system allows a controlling and adjusting of fluid flow, pressure, and/or temperature in the pad.

In doing so, application of the pad and use of the pad may be more convenient and more efficient and effective for the patient, thus enhancing the healing.

In one embodiment, once the treatment starts, the thermal treatment device will start writing data onto the data storage tag (310). Once the treatment stops, and the user moves to another location, the therapeutic pad (10) will be connected to a new thermal treatment device. The latter will read the information from the data storage tag (310), and by doing so it will be able to implement the right treatment protocol without needing any manual input.

Also, because the thermal treatment device will also log on the data storage tag (314) information related to the therapeutic pad (10) performance, usage, quality, etc., the thermal treatment device will be able to control and regulate the use of expired or non-performing therapeutic pads (10).

Preferably, the therapeutic pad (10) is a disposable unit which is replaced after a pre-determined amount of time, due to treatment protocols, safety, or expiry of the pad. The data storage tag (310) allows the treatment device to disable the pad (10) after a pre-determined amount of time, for example, 10 hours of treatment. At that time, it is anticipated that information from the data storage tag (310) on the therapeutic pad (10) is copied to the replacement therapeutic pad (10), via means of an intermediary device, for example, but not limited to the thermal treatment device .

Additionally or alternatively, information can also be copied onto a storage device held on the patient's person, for example, onto an RF bracelet, for example. For example, such information may be uploaded to a computer network or the Internet, either by the treatment device or from the therapeutic pad (10) preferably using wireless transmission means. Such alternative embodiments advantageously allow data specific to the patient to be collated for various purposes, such as but not limited to, generating or modifying treatment protocols, assessing the current status of the patient on their path to recovery, or collecting data in the process of a clinical trial.

Finally, one of the problems with the kind of therapeutic pad (10) described and the domain of the treatment, is the risk of infections due to improper use of the therapeutic pads (e.g. multiple patient use). The operator is able now to register the patient's name, and possibly also to print it and stick it to the therapeutic pad (10). Because the thermal treatment server will ask confirmation of the current patient - therapeutic pad (10) relationship, there will be a further step of control in ensuring the single patient usage of the therapeutic pad (10).

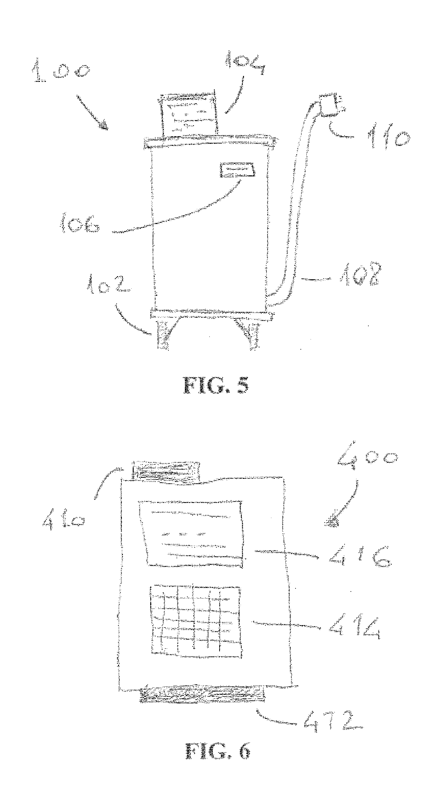

Fig. 5 schematically shows a medical treatment device (100) for controlling a therapeutic pad. The device may or may not have wheels (102) or other means for facilitating the transportation and handling, so that the device is a portable device. The treatment device (100) is equipped with a hardware and software user interface (104). The user interface (104) may be a touch screen user interface, but any other kind that allows the operator of the treatment device (100) to perform and view the input and request and view the output of data and instructions is possible (included but not limited to hardware keyboard, pointing device, audio input and output). Further, the treatment device (100) can have a printer device, embedded (106) or external (connected through radio or wire to the treatment device (100)), for the printing of labels and reports. Finally, the treatment device (100) may be a thermal treatment device and may be equipped with a hydraulic tube (108) and a hydraulic connector (110) to allow the circulating fluid to reach a therapeutic pad through the hydraulic device (200) described in Fig. 2. The hydraulic connector (110) can be formed by two separate hydraulic connectors, or the two connectors can be embedded in a single element, as depicted in Fig. 5.

Fig. 6 schematically shows an (optional) handheld device (400) equipped with a hardware and software user interface (414,416). The user interface (414,416) depicted in figure 6 is a video display (416) with a hardware keyboard (414), but any other kind that allows the operator of the (thermal) treatment device (100) to perform and view the input and to request and view the output of data and instructions is possible (included but not limited to hardware keyboard, pointing device, audio input and output). Further, the handheld device (400) can have a printer device, embedded (412) or external (connected through radio or wire to the handheld device (400)), for the printing of labels and reports. Finally, the handheld device (400) may have a RF reader / writer module (410).

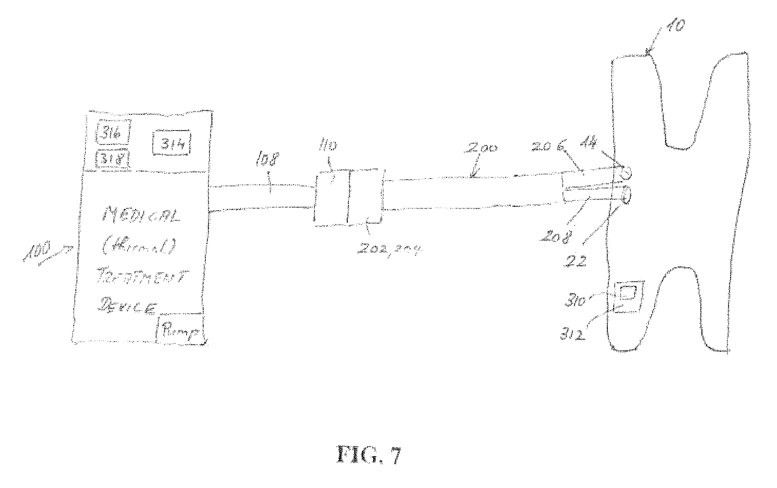

Fig. 7 shows the overall system with the arrangement of the medical treatment device (100) and the pad (10) connected to the device (100) via a tube system (108, 110, 202, 204, 206 and 208).

While the invention has been illustrated and described in detail in the foregoing description, such illustration and description are to be considered illustrative or exemplary and non-restrictive; the invention is thus not limited to the disclosed embodiments. Features mentioned in connection with one embodiment described herein may also be advantageous as features of another embodiment described herein without explicitly showing these features. Variations to the disclosed embodiments can be understood and effected by those skilled in the art and practicing the claimed invention, from a study of the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures can not be used to advantage.

## Claims

1. Therapeutic pad for attachment to the human or animal body, comprising means (312, 310) for storing data.

2. Pad of claim 1, wherein the pad is adapted to provide a thermal energy exchange to the human or animal body.

3. Pad of claim 1 or 2, wherein the pad (10) is adapted to be connected to a hydraulic device (200, 108), particularly via a tube.

4. Pad of claim 1, 2 or 3, further comprising internal fluid communication channels adapted to be externally connected to a tube (212, 214, 108).

5. Pad of one of the preceding claims, wherein the means for storing data is an electronic memory chip.

6. Pad of claim 5, wherein the memory chip is adapted to contactlessly communicate with an external processing unit (316).

7. Pad of claim 5 or 6, wherein the memory chip is attached to an outer or an inner surface of the pad (10).

8. Pad of claim 7, wherein the memory chip (310) is attached to the pad via a tag, particularly via a RF tag (312).

9. Pad of any one of claims 5 to 8, wherein the memory chip is readable and/or writable by more than one pre-determined processing units (316).

10. Pad of any one of the preceding claims, wherein the data is a medical protocol information and/or a result of a medical treatment and/or an information relating to the performance and/or lifetime of the pad.

11. Pad of any one of the preceding claims, wherein the pad is disposable.

12. Therapeutic system comprising:
a therapeutic pad of any one of claims 1 to 11; and
a hydraulic device connected to the pad via a tube (108, 200) for circulating a liquid.

13. System of claim 12, wherein the hydraulic device and the pad are non-removably connected to each other.

14. System of claim 12 or 13, wherein the hydraulic device is controllable by means of a medical treatment device.
